Europäisches Patentamt

European Patent Office

Office européen des brevets

(11) Publication number: **0 205 051**
**B1**

# EUROPEAN PATENT SPECIFICATION

(45) Date of publication of patent specification: **24.05.89**

(51) Int. Cl.⁴: **A 61 K 37/36,** A 61 K 47/00, A 61 K 9/06

(21) Application number: **86107343.5**

(22) Date of filing: **30.05.86**

(54) **A pharmaceutical composition in the form of a cream for dermal and ophthalmic use.**

(30) Priority: **30.05.85 IT 2097085**

(43) Date of publication of application:
**17.12.86 Bulletin 86/51**

(45) Publication of the grant of the patent:
**24.05.89 Bulletin 89/21**

(84) Designated Contracting States:
**AT BE CH DE FR GB IT LI LU NL SE**

(56) References cited:
**EP-A-0 140 998**

**CHEMICAL ABSTRACTS, vol. 101, no. 6, 6th August 1984, page 326, abstract no. 43621z, Columbus, Ohio, US; & JP - A - 59 65 020 (84 65 020) (J C R K.K.) 13-04-1984**

(73) Proprietor: **ZAMBON S.p.A.
Via della Chimica, 9
I-36100 Vicenza (IT)**

(72) Inventor: **Gazzaniga, Annibale
via Generale Porro, 22
I-20027 Rescaldina (Milano) (IT)**
Inventor: **Stroppolo, Federico
via Vedreggio, 17
CH-6963 Pregassona (CH)**
Inventor: **Gianesello, Valter
Palazzo Bellavista via S. Chiesa
CH-6833 Vacallo (CH)**

(74) Representative: **Weinhold, Peter, Dr. et al
Patentanwälte Dipl.-Ing. G. Dannenberg Dr. P.
Weinhold Dr. D. Gudel Dipl.-Ing. S. Schubert Dr.
P. Barz Siegfriedstrasse 8
D-8000 München 40 (DE)**

Courier Press, Leamington Spa, England.

## Description

The present invention relates to a pharmaceutical composition in the form of a cream for dermal and ophthalmic use containing as active ingredient the compound known as EGF.

It is named EGF (from Epidermal Growth Factor) a natural protein existing both in human and in mammals which has a stimulating activity on the cell proliferation of epithelial tissue and so accelerates the healing of ulcers of various origin.

At first EGF was identified and isolated from the mouse submaxillary glands [J. Biol. Chem., *237*, 1555, (1962)].

The analysis of the protein showed the presence of 53 aminoacids with three disulfide bonds. Generally this protein is indicated as mouse-EGF or more shortly m-EGF.

The different methods for separation and purification of m-EGF or some suitable enzymatic treatments can bring to obtain a protein in which 2 or 5 terminal aminoacids are loosed [J. Biol. Chem., *247*, 7609, (1972)]. These derivatives, which are called respectively "EGF-2" and "EGF-5", showed analogous properties to EGF with 53 aminoacids.

EGF is also present in different human biological liquids such as plasma, saliva, urine, amniotic liquid and milk.

Human EGF ("human EGF" or more shortly" "h-EGF") is a protein having substantially the same chemical-physical characteristics and biological activity of m-EGF.

For these reasons from now on the term EGF includes m-EGF as well as EGF-2, EGF-5 and h-EGF or mixtures thereof.

The stimulating activity on the proliferation of epithelial tissue has suggested the use in human therapy to enhance epidermal and corneal healing for instance in case of ulcers and oculistic surgery.

EGF is a very unstable protein (in aqueous solution it degrades for 50% in about two months) and can be preserved only in a freeze-dried form from which a suitable pharmaceutical form is prepared at the time of use.

For these reasons the above use in therapy can only be performed in hospitals.

If we consider the biological activity of EGF, its harmlessness and complete local tolerability as a natural protein, it would be useful to have available a pharmaceutical preparation for dermal or oculistic use to be found in apothecary's shops and therefore employable also out of hospital.

Generally many months pass from the industrial preparation of a drug to its use in therapy, so the practical use of a drug is seldom compatible with a stability lower than one year.

The Health Laws of many Countries impose that the degradation of the active ingredient is lower than 10% during the period of validity of the drug declared by the producer.

Possible pharmaceutical forms of EGF such as aqueous solutions, physiological solutions, saline aqueous solutions or ointments (solid active ingredient dispersed in a fatty phase) such as for instance the formulations described in the Japanese Patent Application published under the number 59/65020 (Chemical Abstracts, vol. 101:43621z) showed not to be suitable in our experiments to preserve EGF for a long period of time. After two months these pharmaceutical forms already show a 20—40% degradation of the active ingredient.

We have now surprisingly found that the pharmaceutical compositions below specified in the form of a cream (oil in water emulsion) ready for use are able to give a remarkable stability to the pharmaceutically active ingredient (EGF) so that, after two years its degradation is lower than 10%.

Object of the present invention are therefore pharmaceutical compositions in the form of a cream for dermal or ophthalmic use having essentially the following composition (w/w):

| | |
|---|---|
| EGF | 0.0001—0.005% |
| surface active agents | 1—10% |
| fatty substances | 5—45% |
| preservatives | 0.3—0.8% |
| purified water | up to 100% |

Within the above values, the fatty substances are preferably employed in amounts between 15 and 35% w/w.

As above mentioned the term EGF includes m-EGF as well as EGF-2, EGF-5 and h-EGF.

The employable surface active agents in the compositions object of the invention are preferably non-ionic surface active agents such as polyoxyethylated aliphatic long chain alcohols, fatty acids and the respective glycerides and sorbitol derivatives or their mixtures.

Particularly, sorbitol derivatives include two classes of surface active agents known as "Span" and "Tween" (both trademarks of ICI Americas) for esters of sorbitol with fatty acids and for the corresponding polyoxyethylated products respectively (Merck Index, 10th edition, No. 8578 page 1250 and No. 7455 page 1095).

For instance, the compounds mainly constituted by sorbitan-monolaurate ("Span 20"), sorbitan-monostearate ("Span 60") and sorbitan-trioleate ("Span 85") belong to the Span group; the compounds mainly constituted by polyoxyethylene sorbitan-monolaurate with on the average 20 mols of ethlenoxyde each mol of substrate ("Tween 20"), polyoxyethylene 20 sorbitan-monostearate ("Tween 60"), polyoxyethylene 20 sorbitan-trioleate ("Tween 85") belong to the Tween group.

Similar products are "Span 40", "Span 65", "Tween 40" and "Tween 65".

For a reference to the surface active agents "Span" and "Tween" see "USAN and the USP Dictionary of Drug Names", Ed. 1980—The United States Pharmacopeial Convention Inc.—Rockville Md.

Mixtures of the surface active agents "Span" and "Tween" showed to be particularly suitable for the employment in the compositions object of the invention.

For this reason and also in view of the fact that the surface active agents known as "Span" and "Tween" are approved as additives for pharmaceutical use in many Countries, the compositions containing a "Span" and "Tween" mixture as a surface active agent in the above amounts represent a preferred embodiment of the present invention.

The amount of surface active agent within the above range (1—10% w/w) and the specific product will be selected so as to ensure a complete emulsification of the fatty phase into the aqueous phase.

The employable fatty substances for the compositions object of the invention are selected among saturated semi-solid or liquid hydrocarbons, saturated or unsaturated fatty acids and their triglycerides, long chain aliphatic alcohols, vegetable or animal waxes and their mixtures.

Examples of specific fatty substances are vaseline, triglycerides of caprylic and caproic acids, cetyl alcohol, stearic alcohol, lanolin and paraffin.

Preservatives are used only to prevent the microbial growth and in view of the fact that generally preservative additives are required by the Pharmacopeia of several Countries for every pharmaceutical preparation in the form of a cream and in a multidose pack.

Some examples of suitable preservatives are
— phenols
— benzylic alcohol
— alkyl-p · hydroxybenzoate and salts thereof
— monothioglycerol
— thimerosal
— benzethonium chloride
— chlorobutanol
— dehydroacetic acid sodium salt
— imidazolidinyl urea and derivatives
— benzyl-alkylammonium chloride
— p · chlorophenol
— p · tert-butylphenol
— cerium III nitrate
— cetyl-alkyl-ammonium chloride
— cetyl-diethylmethyl-ammonium bromide
— chlorothymol
— cresols
— sodium benzoate.

It is important to underline the fact that the compositions object of the invention provide a remarkable stabilisation of the active ingredient even if they do not contain suitable "ad hoc" compounds to stabilize pharmaceutical compositions.

Infact, in the compositions object of the invention it is not required the use of neither anti-oxidizing agents (ascorbyl palmitate, ascorbic acid, vitamin E, butylhydroxyanisole and so on) or protein stabilizers (albumin, aminoacids, inorganic salts, ionic surface active agents).

The preparation of the compositions object of the invention does not require any specific expedient.

A suitable procedure consists in melting (70—80°C) the fatty substance with the surface active agent and then in incorporating still under heating most of the water (pure according to Pharmacopoeia, or sterile for injection in case of an opthalmic cream).

The emulsion is then cooled under stirring up to 25—30°C and an aqueous solution of EGF and preservatives is incorporated in it under stirring.

The cream is further homogenated for instance by a three roller mill and then distributed in tubes.

The preparation of the sterile ophthalmic cream is carried out in a similar way by previously sterilizing the different components and by operating in a sterile environment.

A specific example of a cream for dermal use has the following composition:

| | |
|---|---|
| EGF | 0.0005% (w/w) |
| surface active agents | 4.6% (w/w) |
| fatty substances | 30.6% (w/w) |
| preservatives | 0.4% (w/w) |
| purified water | up to 100% |

As far as the ophthalmic use is concerned, generally creams with a higher concentration of EGF (0.001—0.05%) are preferred in view of the lower amount of cream which is used, the amount of fatty substance is reduced (5—25%) to lower the amount of surface active agent (1—3%) and to make more flowing the pharmaceutical form.

A specific example of a cream for ophthalmic use (useful also for dermal use) is the following:

| | |
|---|---|
| EGF | 0.001% (w/w) |
| surface active agents | 2.8% (w/w) |
| fatty substances | 18.5% (w/w) |
| preservatives | 0.4% (w/w) |
| purified water | up to 100% |

It is known that EGF, and particularly h-EGF, has a chromatographic map made by several peaks. We define as time-stability of EGF not only the conservation of both the biological titre and weight but also the substantial constancy of the respective ratio between the peaks of the chromatographic map.

This last feature has a particular importance as only the constancy of the chromatographic map ensures that there are no modification of the active ingredient and therefore of its characteristics of activity and safeness in the time.

The EGF stability in the cream object of the invention in comparison with its stability in other pharmaceutical forms (aqueous solutions with salts, buffers, albumin, ionic or non-ionic surface active agents and ointments) was evaluated by an indirect determination [based on the EGF interaction with a specific antiserum by Radioimmunoassay (RIA) according to the methodology described in J. Clin. End. and Metab., 45, 1144, (1977)] as well as by a direct determination based on a HPLC method made ready by us and described into details in example 5.

HPLC analysis allows to evaluate both weight variation of the active ingredient and possible variation of its chromatographic map. The EGF concentrations obtained by RIA and HPLC proved to be substantially the same.

By these experiments (see example 5) it is possible to conclude that the creams object of the invention are able to stabilize EGF during a period of two years at least with a degradation lower than 10% and a substantial conservation of the chromatographic map.

Of course this allows to realize a pharmaceutical form having the suitable characteristics of stability for its sale in apothecary's shops too.

The other tested compositions show a degradation of the active ingredient between 15 and 50% already after two months.

Tolerability tests on the creams object of the invention (rabbit eye irritation test, albino guinea pig epidermal sensibilization test, carried out according to regulations accepted by European Pharmacopoeia and by Food and Drug Administration) have shown that these creams are perfectly tolerable and do not cause any irritation.

The activity tests on rabbit have shown that EGF in the preparation in the form of a cream according to the present invention ·has the same activity on the rehabilitation of scared skin as EGF in an extemporaneous aqueous solution formed at the time of use.

The above features are valid also for compositions in the form of a cream stored for two years.

By the creams object of the invention it is possible the therapeutic treatment of several clinical situations such as skin abrasions, surgical·superficial wounds, crural ulcers, decubitus sores, chronic and post-traumatic sores and also, in ophthalmic field, superficial corneal acute or chronic ulcers of contagious origin, corneal burns by chemical or physical agents, corneal ulcers or abrasion by trauma or by external agents, corneal invasion and elcoma, corneal wounds of surgical origin.

The amount of cream to be administered will be generally between 0.5 and 5 g/day and will charge according to different factors such as the seriousness of the ulcer or the wound, its location and extent and, the subjective answer to the treatment.

In order to better illustrate the invention the following examples are given.

Example 1
Preparation of a cream for dermal use.

According to the below reported procedure 10 kg of a cream for dermal use having the following composition are prepared:

| | |
|---|---|
| m-Epidermal Growth Factor | 0.050 g |
| vaseline | 1,200 g |
| "Dermol 1" | 1,200 g |
| cetyl alcohol | 350 g |
| stearyl alcohol | 310 g |
| "Tween 60" | 230 g |
| "Span 60" | 230 g |
| "Conservante G" | 30 g |
| "Antimuffa P" | 10 g |
| purified water | up to 10,000 g |

wherein

"Dermol 1": commercial name by Esperis Society for triglycerides of capric and caprylic acids

"Conservante G": commercial name by Esperis Society for imidalozidinyl urea

"Antimuffa P": commercial name by Esperis Society for sodium dehydroacetate.

Preparation:

An emulsion is prepared by heating at 80°C vaseline and "Dermol 1" and by adding cetyl alcohol, stearyl alcohol, "Tween 60" and "Span 60" under stirring at the same temperature.

The required amount of purified water (except 300 ml) preheated at the same temperature is added to the mixture.

The emulsion is slowly cooled under stirring up to 30°C.

Separately an aqueous solution (300 ml) of EGF, "Conservante G" and "Antimuffa P" is prepared. This solution is added under stirring to the emulsion at the same temperature of 30°C.

The resulting cream is cooled at the temperature of 25°C and further homogenated by a three roller mill.

Then the cream is distributed in 15 g holding tubes.

Example 2

Preparation of a cream for ophthalmic use.

10 kg of a cream for ophthalmic use having the below reported composition are prepared in a sterile environment for presterilized ingredients according to the procedure described in example 1.

| | |
|---|---|
| m-Epidermal Growth Factor | 0.100 g |
| vaseline | 728 g |
| "Dermol 1" | 728 g |
| cetyl alcohol | 212 g |
| stearyl alcohol | 188 g |
| "Tween 60" | 140 g |
| "Span 60" | 140 g |
| "Conservante G" | 36 g |
| "Antimuffa P" | 12 g |
| purified water | up to 10,000 g |

Example 3

Preparation of a dermal cream.

According to the below reported procedure 10 kg of a cream for dermal use having the following composition are prepared:

| | |
|---|---|
| m-Epidermal Growth Factor | 0.05 g |
| stearyl alcohol | 500 g |
| vaseline | 200 g |
| "Solulan L-575" | 200 g |
| "Span 20" | 300 g |
| "Tween 20" | 300 g |
| "Antimuffa P" | 10 g |
| "Conservante G" | 30 g |
| water | up to 10,000 g |

"Solulan L-575": trademark of Amerchol Society for highly purified and water soluble lanolin. The product is a 50% aqueous solution.

Preparation:

A mixture of stearyl alcohol, vaseline, "Solulan L-575" and "Span 20" is heated at 70°C.

The required amount of water (except 300 ml) preheated at the same temperature and containing "Tween 20", "Antimuffa P" and "Conservante G" is added under stirring to this mixture.

The emulsion is slowly cooled under stirring up to 30°C.

Separately an aqueous solution of EGF is prepared. This solution is added to the emulsion under stirring at the same temperature of 30°C.

The resulting cream is further cooled and distributed into suitable tubes.

Example 4

Preparation of an ophthalmic cream.

According to the below reported procedure 10 kg of a cream for ophthalmic use having the following composition are prepared

| m-Epidermal Growth Factor | 0.1 g |
| liquid paraffin | 1,200 g |
| vaseline | 850 g |
| "Tween 40" | 306 g |
| "Span 40" | 154 g |
| cetyl alcohol | 350 g |
| stearyl alcohol | 310 g |
| "Conservante G" | 10 g |
| "Antimuffa P" | 30 g |
| sterile water | up to 10,000 g |

Preparation:

A mixture of liquid paraffin, vaseline, "Tween 40" and "Span 40" is melted at 85°C. A solution made by water (except 300 ml), "Conservante G" and "Antimuffa P" preheated at 85°C is added under stirring to the melted mixture.

The emulsion is slowly cooled under stirring up to the temperature of about 30°C.

Separately an aqueous solution (300 ml) of EGF is prepared. The solution is added under stirring to the emulsion at the temperature of 30°C.

The resulting cream is homogenated and distributed in tubes.

The preparation of the ophthalmic cream is made with sterile materials and in a sterile environment.

Example 5

Determination of EGF stability in the creams object of the invention in comparison with different compositions.

The stability of the active ingredient was evaluated by determining the percentage of the active ingredient present after a prefixed period of time with respect to the starting amount.

The amount of EGF in the different compositions was evaluated by the method of Radioimmunoassay (RIA) described on J. Clin. End. and Metab., *45*(6), 1144, (1977) and by the high pressure liquid chromatography (HPLC) method below described.

HPLC method:

Chromatographic conditions:

Liquid chromatography Hewlett-Packard 1084 B with an UV detector at 215 nm

Column: LC 18DB, 5 mcm, 150 mm×4.6 mm

Eluent A: buffer pH=2.1 (6.1 ml $HClO_4$ in 1000 ml $H_2O$ brought to pH=2.1 with NaOH 2.5 N) Eluent B: acetonitrile

Flow: 1.5 ml/min.

%B: it changes according to a linear gradient which brings the percentage of B from 24% (t=0) to 44% (t=20 min.).

Column temperature: +25°C.

Solvent temperature: +25°C

Standard solution: 2 mg of EGF as standard raw material at known titre are weighed exactly and are diluted up to 10 ml with water. 50 mcl are injected into the chromatograph.

Sample solution by a cream: In a centrifuge test tube 10 g of cream are weighed exactly and the test tube is kept in a water-bath at +80°C for 30 minutes. Then it is centrifuged for 30 minutes at 20,000 r.p.m.

The separated aqueous phase is drawn and filtered on Sep Pack C18, then transferred into an ultrafilter with a membrane with molecular cut off 1000 Dalton by diluting with $H_2O$ up to 50 ml.

The solution is dialyzed 3 times [diluting every time the concentrated (about 10 ml) with $H_2O$ in ratio 1:5].

The concentrated (about 1.5 ml) is withdrawn and diluted up to 2 ml with $H_2O$. 200 mcl of this solution are injected into the chromatograph.

Sample solution by a composition in the form of aqueous solution:

The direct injection of 50 mcl of solution (corresponding to theoretic 10 mcg of EGF) is performed.

Sample solution by a composition in the form of an ointment:

Take 5 g of the ointment (containing theoretically 50 mcg of EGF) and add 20 ml of water; emulsionate the mixture, then heat it at 80°C for 15 minutes. A separation between the aqueous phase (containing EGF) and the fatty phase is obtained. Transfer the aqueous phase into an ultrafilter with a membrane with a molecular cut off 1000 Dalton by diluting up to 50 ml with water.

Dialyze the solution 3 times [by diluting every time the concentrated (about 10 ml) with water in ratio 1:5].

Withdraw the concentrated (about 1.5 ml) and dilute it up to 2 ml with water. 200 mcl of this solution are injected into the chromatograph.

6

The measured amount of EGF in the different samples ($X_c$) expressed in g $10^{-6}$ (micrograms) is determinated by a comparison among the areas according to the following equation:

$$X_c = \frac{A_c}{A_s} \cdot P_s \cdot F_d$$

wherein

$A_c$ = EGF area in the sample

$A_s$ = EGF area in the standard solution

$P_s$ = weight of the standard in g $10^{-6}$

$F_d$ = dilution factor (0.05 for cream and ointment, 1 for aqueous solution).

The $X_c$ value divided by the weight of the sample expressed in grams gives the amount of EGF in 1 g of sample (cream or ointment).

The difference to 100 of the percent ratio between the calculated amount of EGF in the sample at the time t and the initial amount considered as 100% gives the percentage of EGF degradation in the sample at the time t.

Stability tests:

The compositions in the form of a cream described in the examples 1 and 2 were compared with some reference compositions to evaluate the variation of the amount of EGF (m-EGF) in the time.

Reference compositions (all the aqueous solutions contain 200 µg/ml of EGF):

R-1: EGF in aqueous solution (purified water)

R-2: EGF in aqueous solution containing 500 µg/ml of albumin

R-3: EGF in physiologic solution of NaCl (0.9%)

R-4: EGF in isotonic solution containing NaCl, KCl and $CaCl_2$

R-5: EGF in isotonic solution containing NaCl, KCl, $CaCl_2$, $KH_2PO_4$ e $MgSO_4$

R-6: EGF in aqueous solution containing "Tween 60" (23 mg/ml)

R-7: EGF in aqueous solution containing "Tween 60" and "Span 60" in ratio 1:1 (in all 46 mg/ml) R-8: EGF in aqueous solution containing sodium lauryl sulphate (20 µg/ml)

R-9: EGF in aqueous solution containing sodium lauryl sulphate (20 µg/ml)

R-10: EGF in buffered aqueous solution at pH 7 (buffer: $KH_2PO_4/K_2HPO_4$ 0.05 M)

R-11: EGF dispersed in a fatty phase (ointment) having the following composition (w/w):

| | |
|---|---|
| m-EGF | 0.001% |
| liquid paraffin | 10% |
| lanoline | 10% |
| vaseline | up to 100% |

R-12: EGF ointment containing surface active agents having the following composition (w/w):

| | |
|---|---|
| m-EGF | 0.001% |
| "Span 85" | 1.5% |
| "Tween 85" | 3.5% |
| liquid paraffin | up to 100% |

For uniformity, every reference composition was additioned with the same preservatives employed in the compositions of the examples 1 and 2 in corresponding amounts.

The choice of the reference compositions, which are clear for the man skilled in the art, can be so summarized:

The composition R-2 contains albumin which is known generally to have stabilization properties on protein in aqueous solution.

The compositions R-3, R-4, R-5 and R-10 represent different isotonic or buffered physiologic solutions in which it is generally known that proteins are stabilized.

The compositions R-6 and R-7 contain the surface active agents employed also in the cream object of the invention and the compositions R-8 and R-9 contain surface active agents of different kind.

The composition R-11 and R-12 contain fatty substances and particularly R-12 is different conceptually from the compositions according to the present invention only for the lack of water (an ointment instead of a cream).

The stability of the active ingredient was determined by the above described RIA and HPLC methods.

The results are reported in the following tables 1 and 2.

TABLE 1

Titre (%) in EGF of the standard compositions at the time t (every datum results from the average of three tests, the HPLC degradation was calculated as the difference to 100 of the HPLC titre).

| | Titre | | | | Degradation at t=60 days HPLC |
|---|---|---|---|---|---|
| | at t=0 | | at t=60 days | | |
| | HPLC | RIA | HPLC | RIA | |
| R-1 | 100% | 100% | 65% | 57% | 35% |
| R-2 | 100% | 100% | 70% | 71% | 30% |
| R-3 | 100% | 100% | 50% | 53% | 50% |
| R-4 | 100% | 100% | 79% | 80% | 21% |
| R-5 | 100% | — | 75% | — | 25% |
| R-6 | 100% | 100% | 70% | 64% | 30% |
| R-7 | 100% | 100% | 57% | 62% | 43% |
| R-8 | 100% | — | 72% | — | 28% |
| R-9 | 100% | — | 70% | — | 30% |
| R-10 | 100% | — | 85% | — | 15% |
| R-11 | 100% | 100% | 82% | 78% | 18% |
| R-12 | 100% | 100% | 65% | 72% | 35% |

TABLE 2

Titre (%) in EGF of the compositions of the examples 1 and 2 at the time t (every datum is the average of three tests each of them carried out on three different cream preparations having the same composition; the HPLC degradation was calculated as the difference to 100 of HPLC titre)

| Composition | Time | Titre HPLC | Titre RIA | Degradation (HPLC) |
|---|---|---|---|---|
| Example 1 | t=0 | 100% | 100% | — |
| | t=60 days | 99% | 100% | 1% |
| | t=1 year | 96.5% | 97.5% | 3.5% |
| | t=2 years | 93% | 94.5% | 7% |
| Example 2 | t=0 | 100% | 100% | — |
| | t=60 days | 100% | 99% | 0 |
| | t=1 year | 98% | 96% | 2% |
| | t=2 years | 93% | 92% | 7% |

By the data reported in the tables 1 and 2 are following conclusions can be drawn:
— the reference compositions are not suitable for use other that of an extemporaneous preparation. In fact, already after 60 days the active ingredient (EGF) has undergone a degradation higher than 10% and in many cases higher than 30%;

— the compositions in the form of a cream object of the invention show a surprising stabilization of EGF which, after 60 days, has degraded in negligible amounts, if any, and, after two years, it has undergone a degradation lower than 10%;

— the association of the components of the creams object of the invention has a peculiar role in stabilizing EGF. In fact neither the surface active agent in water (R-6 and R-7) nor the fatty phase only (R-11) or, least of all, the fatty phase additioned with a surface active agent (R-12) are able to ensure a sufficient stabilization of the active ingredient.

Example 6

Tolerability tests

By the creams of the examples 1 and 2 the following tests, by the indicated methods, were performed:

— eye irritation on rabbit according to "Recommended Guidelines for Acute Eye Irritation Testing" by "Interagency Regulatory Liaison Group Testing Standards and Guidelines Work Group" on January 1981;

— primary skin irritation on rabbit according to the procedure of Draize described in "Appraisal of the Safety of Chemicals in Foods, Drugs and Cosmetics"—Acute Toxicity Assoc. of Food and Drug Officials of the United States—pages 46—59;

— epidermal sensibilization on albino guinea pig according to an adaptation of the procedure of Maguire described on "The bioassay of contact allergenes in the guinea pig", J. Soc. Cosmet. Chem., *24*, 151, (1973).

The eye and primary dermal irritation tests performed on rabbit by the pharmaceutical preparations in the form of a cream described in the examples 1 and 2 showed that the two tested preparations do not have any irritant property.

The epidermal sensibilization tests performed on albino guinea pig by application of the pharmaceutical preparations in the form of a cream described in the examples 1 and 2, allowed to conclude that the tested preparations responded negatively to sensibilization test. In fact no alteration between the observations made before and after the rousing treatment was observed in any of the tested animals.

Example 7

Activity tests.

The activity of rehabilitation of scared skin of the composition described in example 1 was evaluated in comparison with the activity of an extemporaneous aqueous solution of EGF prepared at the time of use.

The test was carried out on four groups, each of 8 male New Zealand White Rabbits to which an area of $20 \times 6$ cm were previously scared.

One group of animals has not undergone any treatment and was used as control, a second group was treated with a placebo cream, a third group was cutaneously treated with 4 g/die/animal of the composition in the form of a cream described in example 1 (EGF concentration=5 µg/g) and a fourth group of animals was cutaneously treated with 4 g/die/animal of extemporaneous aqueous solution of EGF at the same concentration.

The daily amount of placebo, preparation in the form of a cream and extemporaneous solution was administered in two daily portions until "restitutio ad integrum".

The "restitutio ad integrum" and the histopathologic examination were the studied parameters.

The statistical analysis of the results (Wilcoxon-Brealow test) allowed to show that, in the rate of rehabilitation of scared skin, exists a significant difference among the check animals or the animals treated with placebo and the animals treated with the pharmaceutical preparations in the form of a cream described in example 1.

On the contrary, there is no significant difference between the animals treated with the cream of the example 1 and those treated with the extemporaneous solution of EGF.

It follows that the pharmaceutical compositions in the form of a cream according to the present invention, besides ensuring a high stability to the active ingredient, do not affect the pharmaceutical activity thereof.

**Claims**

1. A pharmaceutical composition in the form of a cream consisting of

| EGF | 0.0001—0.005% (w/w) |
|---|---|
| surface active agent | 1—10% (w/w) |
| fatty substances | 5—45% (w/w) |
| preservatives | 0.3—0.8% (w/w) |
| purified water | up to 100% |

2. A pharmaceutical composition according to claim 1 in which EGF is chosen among mouse-EGF, EGF-2, EGF-5 and human-EGF or their mixtures.

3. A pharmaceutical composition according to one or both of the claims 1—2 in which the surface active agent consist of a mixture of esters of fatty acids with derivative of sorbitol and the corresponding polyoxyethylated products, respectively.

4. A pharmaceutical composition according to one or more of the claims 1—3 in which the amount of fatty substances is preferably within 15% and 35% w/w.

5. A pharmaceutical composition according to one or more of the claims 1—4 in which the fatty substances are chosen among semi-solid or liquid saturated hydrocarbons, saturated or unsaturated fatty acids and their triglycerides, long chain aliphatic alcohols, vegetable or animal waxes and their mixtures.

6. A pharmaceutical composition according to one or more of the claims 1—4 in which the fatty substances consist of mixtures of saturated or unsaturated semi-solid or liquid hydrocarbons, triglycerides of fatty acids and long chain aliphatic alcohols.

7. A pharmaceutical composition according to one or more of the claims 1—6 in which the preservatives consist of mixtures of imidazolidinyl-urea and sodium dehydroacetate.

8. A pharmaceutical composition according to one or more of the claims 1—7 for ophthalmic use.

9. A pharmaceutical composition according to claim 1 consisting of

| | |
|---|---|
| EGF | 0.0005% (w/w) |
| surface active agents | 4.6% (w/w) |
| fatty substances | 30.6% (w/w) |
| preservatives | 0.4% (w/w) |
| purified water | up to 100% |

10. A pharmaceutical composition according to claim 1 consisting of

| | |
|---|---|
| EGF | 0.001% (w/w) |
| surface active agents | 2.8% (w/w) |
| fatty substances | 18.5% (w/w) |
| preservatives | 0.4% (w/w) |
| purified water | up to 100% |

## Patentansprüche

1. Pharmazeutische Zusammensetzung in Form einer Creme, die besteht aus

| | |
|---|---|
| EGF | 0,0001 bis 0,005% (Gew./Gew.) |
| oberflächenaktivem Mittel | 1 bis 10% (Gew./Gew.) |
| Fettsubstanzen | 5 bis 45% (Gew./Gew.) |
| Konservierungsmitteln | 0,3 bis 0,8% (Gew./Gew.) |
| gereinigtem Wasser | auf 100%. |

2. Pharmazeutische Zusammensetzung nach Anspruch 1, in welcher EGF ausgewählt ist aus Maus-EGF, EGF-2, EGF-5 und Human-EGF oder deren Mischungen.

3. Pharmazeutische Zusammensetzung nach einem oder beiden der Ansprüche 1 bis 2, in welcher das oberflächenaktive Mittel aus einer Mischung von Estern von Fettsäuren mit Derivaten von Sorbit bzw. den entsprechenden polyoxyethylierten Produkten besteht.

4. Pharmazeutische Zusammensetzung nach einem oder mehreren der Ansprüche 1 bis 3, in welcher die Menge an Fettsubstanzen vorzugsweise zwischen 15 und 35% Gew./Gew. liegt.

5. Pharmazeutische Zusammensetzung nach einem oder mehreren der Ansprüche 1 bis 4, in welcher die Fettsubstanzen ausgewählt sind aus halbfesten oder flüssigen, gesättigten Kohlenwasserstoffen, gesättigten oder ungesättigten Fettsäuren und deren Triglyceriden, langkettigen, aliphatischen Alkoholen, pflanzlichen oder tierischen Wachsen und deren Mischungen.

6. Pharmazeutische Zusammensetzung nach einem oder mehreren der Ansprüche 1 bis 4, in welcher die Fettsubstanzen aus Mischungen von gesättigten oder ungesättigten, halbfesten oder flüssigen Kohlenwasserstoffen, Triglyceriden von Fettsäuren und langkettigen, aliphatischen Alkoholen bestehen.

7. Pharmazeutische Zusammensetzung nach einem oder mehreren der Ansprüche 1 bis 6, in welcher die Konservierungsmittel aus Mischungen von Imidazolidinyl-harnstoff und Natriumdehydroacetat bestehen.

8. Pharmazeutische Zusammensetzung nach einem oder mehreren der Ansprüche 1 bis 7 zur ophthalmischen Verwendung.

9. Pharmazeutische Zusammensetzung nach Anspruch 1, die aus

| | |
|---|---|
| EGF | 0,0005% (Gew./Gew.) |
| oberflächenaktiven Mitteln | 4,6% (Gew./Gew.) |
| Fettsubstanzen | 30,6% (Gew./Gew.) |
| Konservierungsmitteln | 0,4% (Gew./Gew.) |
| gereinigtem Wasser | auf 100% besteht. |

10. Pharmazeutische Zusammensetzung nach Anspruch 1, die aus

| | |
|---|---|
| EGF | 0,001% (Gew./Gew.) |
| oberflächenaktiven Mitteln | 2,8% (Gew./Gew.) |
| Fettsubstanzen | 18,5% (Gew./Gew.) |
| Konservierungsmitteln | 0,4% (Gew./Gew.) |
| gereinigtem Wasser | auf 100% besteht. |

**Revendications**

1. Composition pharmaceutique sous la forme d'une crème se composant de:

| | |
|---|---|
| EGF | 0,0001—0,005% (poids/poids) |
| agent tensio-actif | 1—10% (poids/poids) |
| substances grasses | 5—45% (poids/poids) |
| agents de conservation | 0,3—0,8% (poids/poids) |
| eau purifiée | jusqu'à 100%. |

2. Composition pharmaceutique suivant la revendication 1, caractérisée en ce que l'EGF est choisi parmi l'EGF de souris, l'EGF-2, l'EGF-5 et l'EGF humain ou leurs mélanges.

3. Composition pharmaceutique suivant l'une ou l'autre des revendications 1 et 2, caractérisée en ce que l'agent tensioactif se compose d'un mélange d'esters d'acides gras avec respectivement un dérivé de sorbitol et les produits polyoxyéthylés correspondants.

4. Composition pharmaceutique suivant l'une quelconque des revendications 1 à 3, caractérisée en ce que la quantité de substances grasses se situe de préférence entre 15% et 35% en poids/poids.

5. Composition pharmaceutique suivant l'une quelconque des revendications 1 à 4, caractérisée en ce que les substances grasses sont choisies parmi les hydrocarbures saturés semi-solides ou liquides, les acides gras saturés ou insaturés et leurs triglycérides, les alcools aliphatiques à longue chaîne, les cires végétales ou animales et leurs mélanges.

6. Composition pharmaceutique suivant l'une quelconque des revendications 1 à 4, caractérisée en ce que les substances grasses se composent de mélanges d'hydrocarbures semi-solides ou liquides, saturés ou insaturés, de triglycérides d'acides gras et d'alcools aliphatiques à longue chaîne.

7. Composition pharmaceutique suivant l'une quelconque des revendications 1 à 6, caractérisée en ce que les agents de conservation se composent de mélanges d'imidazolidinyl-urée et de déshydroacétate de sodium.

8. Composition pharmaceutique suivant l'une quelconque des revendications 1 à 7, pour application ophtalmique.

9. Composition pharmaceutique suivant la revendication 1, se composant de:

| | |
|---|---|
| EGF | 0,0005% (poids/poids) |
| agents tensio-actifs | 4,6% (poids/poids) |
| substances grasses | 30,6% (poids/poids) |
| agents de conservation | 0,4% (poids/poids) |
| eau purifiée | jusqu'à 100%. |

10. Composition pharmaceutique suivant la revendication 1, se composant de:

| | |
|---|---|
| EGF | 0,001% (poids/poids) |
| agents tensio-actifs | 2,8% (poids/poids) |
| substances grasses | 18,5% (poids/poids) |
| agents de conservation | 0,4% (poids/poids) |
| eau purifiée | jusqu'à 100% |